**Europäisches Patentamt**

**European Patent Office**

**Office européen des brevets**

(11) Publication number: **0 125 764**
**B1**

# EUROPEAN PATENT SPECIFICATION

(45) Date of publication of patent specification: **07.12.88**

(51) Int. Cl.⁴: **C 12 N 1/14, C 12 P 7/64**

(21) Application number: **84301889.6**

(22) Date of filing: **20.03.84**

(54) Method for the preparation of a fungal body and a lipid therefrom.

(30) Priority: **11.05.83 JP 82349/83**

(43) Date of publication of application:
**21.11.84 Bulletin 84/47**

(45) Publication of the grant of the patent:
**07.12.88 Bulletin 88/49**

(84) Designated Contracting States:
**DE FR GB NL SE**

(56) References cited:
**CHEMICAL ABSTRACTS, vol. 98, no. 7, 14th
February 1983, page 518, no. 51917k,
Columbus, Ohio, US; T. YOKOCHI et al.:
"Studies on production of lipids in fungi. X.
Influence of culture conditions on lipid
composition of two strains of Mortierella
isabellina from n-paraffin as a carbon source" &
YUKAGAKU 1982, 31(12), 993-1003**

**CHEMICAL ABSTRACTS, vol. 98, no. 5, 31st
January 1983, page 534, no. 33013h, Columbus,
Ohio, US; O. SUZUKI et al.: "Studies on
production of lipids in fungi. VIII. Influence of
cultural conditions on lipid compositions of
two strains of Mortierella isabellina" &
YUKAGAKU 1982, 31(11), 921-31**

(73) Proprietor: **THE STATE OF JAPAN, as
Represented by the DIRECTOR GENERAL of the
AGENCY of INDUSTRIAL SCIENCE and
TECHNOLOGY
3-1, Kasumigaseki 1-chome Chiyoda-ku
Tokyo 100 (JP)**

(72) Inventor: **Suzuki, Osamu c/o National Chemical
Laboratory
for Industry 1, Higashi 1-chome
Yatabe-machi Tsukuba-gun Ibaraki-ken (JP)**
Inventor: **Yokochi, Toshihiro National Chemical
Laboratory
for Industry 1, Higashi 1-chome
Yatabe-machi Tsukuba-gun Ibaraki-ken (JP)**

(74) Representative: **Bizley, Richard Edward et al
BOULT, WADE & TENNANT 27 Furnival Street
London EC4A 1PQ (GB)**

(56) References cited:
**CHEMICAL ABSTRACTS, vol. 96, no. 5, 1st
February 1982, page 320, no. 31351b,
Columbus, Ohio, US; O. SUZUKI et al.:
"Studies on production of lipids in fungi. II.
Lipid compositions of six species of mucorales
in zygomycetes" & YUKAGAKU 1981, 30(12),
863-8**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

(56) References cited:

CHEMICAL ABSTRACTS, vol. 98, no. 5, 31st January 1983, page 537, no. 33069f, Columbus, Ohio, US; & JP - A - 57 144 986 (AGENCY OF INDUSTRIAL SCIENCES AND TECHNOLOGY) 07-09-1982

CHEMICAL ABSTRACTS, vol. 64, no. 2, 17th January 1966, column 2458b, Columbus, Ohio, US; C.G.C. CHESTERS et al.: "Nutritional factors in relation to growth and fat synthesis in Mortierella vinacea" & J. GEN. MICROBIOL. 41(1), 127-34 (1965)

PROCESS BIOCHEMISTRY, vol. 9, November 1974, pages 14-22; D.A. WHITWORTH et al.: "Microorganisms as a potential source of oils and fats"

J.GEN.MICROBIOL. (1965), vol. 41, pp. 127-134 YUKAGAKU (1982), vol. 31 (11), 921-931

# EP 0 125 764 B1

**Description**

The present invention relates to a method for the preparation of a fungal body containing a large amount of lipid and a method for obtaining a lipid from such a body. More particularly, the invention relates to a method for the preparation of a fungal body of certain fungus stocks, e.g. *isabellina, vinacea, ramanniana* var. *anglispora, nana* and the like belonging to the genus of Mortierella, rich in the content of lipid and a method for obtaining lipid therefrom.

It has been hitherto reported that several fungal bodies contain a considerably large amount of lipid including neutral lipids, i.e. oils and fats, and polar lipids, i.e. phospholipids and glycolipids in a content of, for example, 25 to 65% by weight on the dry basis. Such high-lipid filamentous fungus moulds are exemplified by *Geotricum candidum, Fusarium lini, Fusarium buligenum, Penicillium lilacinum, Penicillium soppi, Penicillium spinulosum, Apsergillus nidulance* and *Mucor sacineleudes* reported by K. Yamada "Shokuhin Kogyo Biseibutsu-gaku (Microbiology in Food Industry)", page 62, *Mortierella vinacea* reported by C. G. C. Chester et al., J. Gen. Microbiol, *41*, 127 (1965) and *Aspergillus terreus, Aspergillus Ochraceus, Cladosporium fulvum, Cladosporium herbarum* and *Penicillium gladioli* reported by J. Singh et al. Fd. Agric., *23*, 1113 (1972).

These fungi can be cultured in a culture medium containing a carbohydrate as the carbon source and the preferable concentration of the carbohydrate as the nutrient in the culture medium is reportedly 20 to 60 g/litre when they are cultured in a flask or in a small-size culture tank. When a fungal body of high lipid content is desired of these fungi, the velocity and extent of the multiplication of the filamentous fungus body are usually not so high that the carbohydrate as the carbon source is not exhaustively consumed up. For example, it is reported by Chester et al. that the best results obtained in the culture of *Mortierella vinacea* were that the use of a culture medium containing glucose in an initial concentration of 21.2 g/litre at 20°C gave 4.7 g/litre of the fungal body as dried containing 3 g/litre of the lipid extractable therefrom after consumption of 17 g of starting cluose for 10 days. When molasses is used as the carbon source in the culture of *Penicillium spinurosum*, it is noted that the increase in the concentration of the carbon source in the culture medium does not contribute to the increase of the multiplication of the fungal body so much with rather decreased proportion of the consumed carbon source on the contrary. In this case, the highest results reported by Khen et al. J. Can, Microbiol., *7*, 895 (1961), are that the amount of the multiplication of the fungal body as dried was 22 g/litre containing only 2.5 g/litre of the lipid extractable therefrom after 6 days of culture at 30°C starting with a molasses concentration of 164 g/litre of which about 40% is consumed by the multiplication of the fungus.

The present invention has been completed on the basis of the discovery that, when cultured under specific culture conditions, certain specific filamentous fungus stocks of *Mortierella isabellina, M. vinacea, M. rammanniana* var. *anglispora* and *M. nana* can give fungal bodies of very high lipid content of 35 to 70% by weight with high productivity. The possibility of such a high-density culture of *Mortierella* fungi has been quite unexpected because it is generally accepted that filamentous fungi multiplying with hyphae can hardly be cultured in a high density condition of the fungal body, different from bacteria and yeast. The present discovery however, shows that these specific fungi can multiply in small discrete units without extending the hyphae in a culture medium containing a carbohydrate as the carbon source under an agitating condition by aeration when the velocity of aeration is increased to give a possibility of obtaining a fungal body of high lipid content in very high density per unit volume of the medium. For example, glucose as the starting carbohydrate contained in the culture medium in a concentration as high as 270 g/litre is exhaustively consumed by the culture of the fungus at 30°C for 72 hours to give 100 g/litre medium or more of the fungal body as dried containing about 50 g/litre of the lipid corresponding to a lipid content of about 50%.

Thus, the method of the present invention for the preparation of a fungal body containing a large amount of the lipid comprises culturing a fungus stock of the species *Mortierella* selected from *M. isabellina* IFO 7824, 7873, 7884, 8183 and 8308, *M. vinacea* IFO 6738, M. *ramanniana* var. *anglispora* IFO 8187 and *M. nana* IFO 8794, under aeration in a liquid culture medium containing a carbohydrate as the carbon source at a concentration of from 60 to 400 g/litre to grow a fungal body containing from 35 to 70% lipid on a total dry weight basis.

Further, the method of the present invention for the preparation of a lipid from a fungal body comprises culturing a fungus stock of *M. isabellina, M. vinacea, M. ramanniana* var. *anglispora* or *M. nana* under aeration in a culture medium containing a carbohydrate as the carbon source in a concentration from 60 to 400 g/litre to grow a fungal body containing a lipid, optionally separating the body from the culture medium, and extracting the lipid contained in the body, e.g. with an organic solvent.

As is understood from the above description, the inventive method is performed by use of the filamentous fungal stocks belonging to the genus of *Mortierella* including *M. isabellina* IFO 7824, 7873, 7884, 8183 and 8308, *M. vinacea* IFO 6738, *M. ramanniana* var. *anglispora* IFO 8187 and *M. nana* IFO 8794. Each of these stocks is kept and available at the Institute for Fermentation, Osaka and also registered in the IFO Catalogue bearing the IFO number as given above.

In culturing the fungal stock, the culture medium should contain a carbohydrate as the carbon source which may be, for example, glucose, fructose, saccharose, molasses, starch, saccharification liquid of wood and others. The concentration of the carbohydrate in the culture medium should be high enough in

3

the range from 60 to 400 g per litre of the culture medium. The nitrogen source to be added to the culture medium may be any one of inorganic nitrogen sources such as ammonium nitrate, ammonium sulphate, ammonium chloride and ammonium phosphate or organic nitrogen sources such as urea, peptone, yeast extract and corn steep liquor. The culture medium should contain small amounts of inorganic metal salts such as potassium dihydrogenphosphate $KH_2PO_4$, dipotassium hydrogenphosphate $K_2HPO_4$, sodium chloride NaCl, iron (II) sulphate $FeSO_4 \cdot 7H_2O$, magnesium sulphate $MgSO_4 \cdot 7H_2O$, zinc sulphate $ZnSO_4 \cdot 7H_2O$ and the like. It is of course optional that certain micronutrients and other nutritive substances may be added to the culture medium according to need.

The culturing of the above mentioned filamentous fungus is performed usually in a liquid medium with agitation by aeration. The value of pH of the culture medium should preferably be in the range from 4.0 to 6.0 and culturing is continued for 2 to 15 days at a temperature in the range from 15 to 40°C under agitation at a relatively high velocity of 300 to 800 r.p.m. with aeration at a rate of 0.5 to 2 v.v.m. In this manner, a fungal body of very high lipid content is produced in the culture medium in a high density so that the body is separated from the culture medium and the lipid contained therein is extracted by a suitable means such as extraction by use of an organic solvent. It is noted that, in accordance with the culturing method of the present invention, the multiplication of the filamentous fungus takes place with relatively suppressed extension of the hyphae in extremely minute discrete units each containing only 1 to 10 cells so that the separation of the fungal body from the culture medium is greatly facilitated by use of, for example, a dehydrator or the like machine to give a body with an advantageously low water content of as low as about 60%. The recovery of the lipid from the thus dehydrated fungal body is undertaken according to a conventional procedure such as extraction with an organic solvent.

The inventive method performed in the above described manner can afford the possibility of the production of a lipid with very high productivity because the culturing of the filamentous fungus can be performed in very high density to give a body of very high lipid content by use of a culture medium containing a carbohydrate as the carbon source in a high concentration hitherto not used in the culture of filamentous fungi along with ease and high efficiency in the recovery of the lipid from the thus grown fungal body.

The advantage in the productivity is particularly apparent from the calculation of the capacity of the apparatus required for unit production of the lipid. For example, the hourly production of the fungal body per unit volume of the culture medium would be 1.7 g body per litre per hour assuming a fungal body multiplication of 100 g/litre medium and in consideration of the culture time necessary to obtain this multiplication estimated from the results of the experimentation described below. Therefore, a constant yearly running of 1000 $m^3$ of the culture medium, which is in practice possible by a combined cycling use of 3 culture tanks each having a capacity of 800 $m^3$, would provide a yearly fungal body production of 14,000 tons/year corresponding to a yearly lipid production of 7,000 tons/year assuming a 50% content of the lipid in the body.

In addition, the lipid produced in this manner contains more than 95% of fats or oils, i.e. triglycerides, so that it is useful not only as a food but also as a starting material for the manufacture of various products derived from fats and oils. The phospholipids and glycolipids contained in the lipid are useful as an ingredient of medicines or as a surface active agent. Furthermore, the fungal body residue after extraction of the lipid is composed mainly of proteins and nucleic acids respectively useful in the applications as a provender and medicines.

The following Examples illustrate the inventive method in more detail but do not limit the scope of the invention in any way.

Example 1

Culture media were prepared each by dissolving in 1000 ml of deionized water a varied amount of a carbon source which was either glucose or molasses, 2 g of potassium dihydrogenphosphate $KH_2PO_4$, 0.3 g of magnesium sulphate $MgSO_4 \cdot 7H_2O$, 0.1 g of sodium chloride NaCl, 0.2 g of malt extract, 0.2 g of yeast extract, 0.1 g of peptone, 10 mg of iron (II) sulphate $FeSO_4 \cdot 7H_2O$, 10 mg of calcium chloride $CaCl_2 \cdot 2H_2O$, 0.2 mg of copper (II) sulphate $CuSO_4 \cdot 5H_2O$, 1.0 mg of manganese sulphate $MnSO_4 \cdot 4H_2O$ and a varied amount of a nitrogen source which was either ammonium sulphate or urea. The formulation of the carbon source and the nitrogen source is shown in Table 1 below. When 60 g of glucose and 3 g of mamonium sulphate were added to the medium as the carbon source and the nitrogen source, respectively, the C/N ratio, i.e. the weight ratio of the carbon atoms to nitrogen atoms, in the culture medium was about 40 although the C/N ratio was varied in some tests to examine the effects of the nitrogen concentration or C/N ratio.

Into a culture tank of 10 litres or 30 litres capacity were introduced 6 litres or 20 litres, respectively, of the above prepared culture medium to which either one of the above named fungal stocks was inoculated and cultured therein at 30°C under agitation, at a velocity of 300 to 700 r.p.m. with aeration at a rate of 0.5 to 2.0 v.v.m. In the course of culturing in this manner, a 100 ml portion of the culture medium was periodically taken out of the tank and filtered to separate the fungal body from the culture medium. A part of the thus collected body was subjected to determination of the water content by drying in a thermostat at 120°C for 24 hours after exact weighing and the remainder was used for the extraction of the lipid. Thus, wet fungal body obtained by filtration was added to a 2:1 by volume mixture of chloroform and methyl alcohol and homogenized in the presence of glass beads to effect simultaneous grinding of the body and extraction of the lipid from the ground body into the solvent. This extraction procedure was repeated 5 times each with a fresh portion of the solvent and all the extract solutions were combined together. After purification by the method of partition washing according to Folch, the solvents were removed from the combined extract solution by evaporation under reduced pressure and the amount of the total lipid was determined by weighing the residual matter.

The culture medium after separation of the fungal body by filtration was subjected to high-performance liquid chromatography (HPLC) to determine the concentration of the unconsumed carbohydrate, i.e. glucose per se or glucose, fructose and saccharose in the molasses. When the results of this high-performance liquid chromatography indicated complete consumption of the carbohyrate in the culture medium, the culturing was terminated and the time from the start was recorded as shown in Table 1.

Table 1 gives the results obtained with *M. isabellina* IFO 7884 and includes the data of the fungal body multiplication as the dried amount in g/litre of the medium, the amount of the lipid recovered therefrom in g/litre, the content of lipid in the dry fungal body in %, the lipid coefficient, i.e. the amount of the produced lipid in g per 100 g of the consumed carbohydrate, and the fungus body coefficient, i.e. the amount of dry fungal body in g per 100 g of the consumed carbohydrate. The data are the results obtained for the final culture medium after complete consumption of the carbohydrate, which was subjected to centrifugal separation of the fungal body.

As is shown in Table 1, the multiplication of the fungal body was rapid even when the concentration of the carbohydrate, i.e. glucose or molasses, was as high as 250 g/litre or more to give a fungal body concentration of about 100 g/litre and a lipid production of about 50 g/litre or more corresponding to a lipid content of 45% or higher in the dry fungal body.

When the starting glucose concentration was increased to 390 g/litre, a final fungal body concentration of 156 g/litre was reached corresponding to a lipid production of 83 g/litre. In this case, however, the velocity of fungal body multiplication was remarkably reduced due to substrate inhibition, especially, at the starting stage of the culturing in comparison with the case where the concentration of the carbohydrate was 300 g/litre or smaller, taking almost twice the culture time for completion of multiplication. Therefore, such a condition of an extremely high carbohydrate concentration is not always practical in view of the excessively long culture time for the multiplication of the fungal body but the final fungal body concentration per se is still so high as not to be achieved in the prior art methods.

TABLE 1

| Experiment No. | 1 | 2 | 3 | 4 | 5 | 6 | 7 | 8 | 9 |
|---|---|---|---|---|---|---|---|---|---|
| Tank capacity, litres | 10 | 10 | 10 | 10 | 10 | 10 | 30 | 30 | 30 |
| Culture time, hours | 60 | 36 | 54 | 54 | 72 | 72 | 72 | 108 | 168 |
| Carbon source[1] | MO | GL | GL | GL | GL | GL | GL | MO | GL |
| Concentration of carbon source, g/litre | 64 | 60 | 135 | 135 | 220 | 220 | 270 | 280 | 390 |
| Nitrogen source[2] | AS | AS | AS | UR | UR | UR | UR | UR | UR |
| Concentration of nitrogen source, g/litre | 3.0 | 3.0 | 7.5 | 3.4 | 5.7 | 8.4 | 10 | 10 | 17 |
| Dry weight of fungal body obtained, g/litre | 23.2 | 22.8 | 49.6 | 45.9 | 77.2 | 80.3 | 103.5 | 104.2 | 156.4 |
| Amount of lipid produced, g/litre | 9.9 | 10.3 | 20.2 | 23.0 | 44.1 | 34.3 | 49.4 | 53.1 | 83.1 |
| Content of lipid, % by weight | 42.7 | 45.1 | 40.7 | 50.0 | 57.1 | 42.7 | 47.7 | 51.0 | 53.1 |
| Lipid coefficient, g/100 g · carbohydrate | 15.5 | 17.2 | 15.0 | 17.0 | 20.0 | 15.6 | 18.3 | 19.0 | 21.3 |
| Fungal body coefficient, g/100 g · carbohydrate | 36.3 | 38.0 | 36.9 | 34.0 | 36.3 | 38.7 | 38.3 | 37.4 | 40.1 |

[1] MO: molasses; GL: glucose
[2] AS: ammonium sulphate; UR: urea.

Example 2

Various kinds of filamentous fungi belonging to the genus of *Mortierella* were cultured in a culture tank of 10 litre capacity under the same culturing conditions as in Example 1 except that the concentrations of glucose as the carbon source and urea as the nitrogen source were 200 g/litre and 6.5 g/litre, respectively, in each of the culture tests. This carbohydrate concentration of 200 g/litre was undertaken in consideration of the fact that the culture test by use of the 10 litre culture tank was under a limitation of the apparatus in respect of the concentration of the dissolved oxygen acting as a rate determining factor on the velocity of the fungus multiplication so that the concentration of the carbon source could not be so high as in the use of the 30 litre culture tank. Nevertheless, the glucose in the culture medium was exhaustively consumed in each of the culture tests as is shown in Table 2 below to give a fungal body multiplication of at least 70 g/litre and at least 30 g/litre of the lipid production regardless of the species of fungi.

TABLE 2

| Experiment No. | 10 | 11 | 12 | 13 | 14 | 15 | 16 |
|---|---|---|---|---|---|---|---|
| Fungus | I | II | III | IV | V | VI | VII |
| Culture time, hours | 72 | 72 | 72 | 72 | 96 | 96 | 96 |
| Dry weight of fungal body obtained, g/litre | 76.3 | 81.6 | 79.8 | 73.2 | 70.3 | 72.5 | 75.7 |
| Amount of lipid produced, g/litre | 34.9 | 32.8 | 33.7 | 35.4 | 36.2 | 33.1[*] | 29.8 |
| Content of lipid, % by weight | 45.8 | 40.2 | 42.2 | 48.3 | 51.5 | 45.6 | 39.4 |
| Lipid coefficient, g/100 g · carbohydrate | 17.5 | 16.4 | 16.9 | 17.7 | 18.1 | 16.6 | 14.9 |
| Fungal body coefficient, g/100 g · carbohydrate | 38.2 | 40.8 | 39.9 | 36.6 | 35.2 | 36.3 | 37.8 |

I: *Mortierella isabellina*, IFO 7824
II: *Mortierella isabellina*, IFO 7873
III: *Mortierella isabellina*, IFO 8183
IV: *Mortierella isabellina*, IFO 8308
V: *Mortierella vinacea*, IFO 6738
VI: *Mortierella nana*, IFO 8794
VII: *Mortierella rammaniana* var. *anglispora*, IFO 8187
[*] In addition, 2.5 g/litre of exofungal lipid were formed.

## Claims

1. A method for the preparation of a fungal body rich in lipid content which comprises culturing a fungus stock of the species *Mortierella* selected from *M. isabellina* IFO 7824, 7873, 7884, 8183 and 8308, *M. vinacea* IFO 6738, M. *ramanniana* var. *anglispora* IFO 8187 and *M. nana* IFO 8794, under aeration in a liquid culture medium containing a carbohydrate as the carbon source at a concentration of from 60 to 400 g/litre to grow a fungal body containing from 35 to 70% lipid on a total dry weight basis.

2. A method as claimed in claim 1, wherein the carbohydrate is glucose or molasses.

3. A method as claimed in claim 1 or claim 2 wherein the culture medium has a pH in the range of from 4 to 6.

4. A method for the preparation of a fungal lipid which comprises effecting a method as claimed in any one of claims 1 to 3, and extracting the lipid contained in the resulting fungal body.

5. A method as claimed in claim 4 wherein the lipid is extracted from the fungal body with organic solvent.

6. A method as claimed in claim 4 or claim 5 wherein prior to lipid extraction the resulting mixture of fungal body and culture medium is dehydrated.

7. A method as claimed in claim 4 including separating the resulting fungal body from the culture medium.

## Patentansprüche

1. Verfahren zur Herstellung eines pilzähnlichen Gegenstandes, reich an Fettgehalt, das umfaßt Kultivieren eines Pilzstamms der Spezies *Mortierella*, ausgewählt aus *M. isabellina* IFO 7824, 7873, 7884, 8183 und 8308, *M. vinacea* IFO 6738, M. *ramanniana* var. *anglispora* IFO 8187 und *M. nana* IFO 8794, unter Luftzufuhr in einem flüssigen Kulturnährboden, enthaltend ein Kohlenhydrat als KOhlenstoffquelle bei einer Konzentration von 60 bis 400 g/Liter zum Züchten eines pilzähnlichen Gegenstandes enthaltend von 35 bis 70% Fett auf einer Gesamttrockengewichtsbasis.

2. Verfahren nach Anspruch 1, bei dem das Kohlenhydrat Glucose oder Melasse ist.

3. Verfahren nach Anspruch 1 oder Anspruch 2, bei dem der Kulturnährboden einen pH im Bereich von 4 bis 6 aufweist.

4. Verfahren zur Herstellung eines pilzähnlichen Fettes, das umfaßt Ausführen eines Verfahrens nach einem beliebigen der Ansprüche 1 bis 3 und Extraktion des im sich ergebenden pilzähnlichen Gegenstand enthaltenen Fettes.

5. Verfahren nach Anspruch 4, bei dem das Fett aus dem pilzähnlichen Gegenstand mit organischem Lösungsmittel extrahiert wird.

6. Verfahren nach Anspruch 4 oder Anspruch 5, bei dem die sich ergebende Mischung aus pilzähnlichem Gegenstand und Kulturnährboden vor der Fettextraktion entwässert wird.

7. Verfahren nach Anspruch 4, einschließend Trennen des sich ergebenden pilzähnlichen Gegenstandes vom Kulturnährboden.

**Revendications**

1. Procédé pour la préparation d'un corps fongique riche en lipide qui comprend la culture d'une souche fongique de l'espèce *Mortierella* choisie parmi *M. isabellina* IFO 7824, 7873, 7884, 8183 et 8308, *M. vinacea* IFO 6738, M. *ramanniana* variété *anglispora* IFO 8187 et *M. nana* IFO 8794, sous aération dans un milieu de culture liquide contenant un glucide en tant que source de carbone à une concentration de 60 à 400 g/litre pour faire croître un corps fongique contenant de 35 à 70% de lipide sur la base du poids sec total.

2. Procédé selon la revendication 1, dans lequel le glucide est le glucose ou la mélasse.

3. Procédé selon la revendication 1 ou la revendication 2, dans lequel le milieu de culture a un pH dans le domaine de 4 à 6.

4. Procédé pour la préparation d'un lipide fongique qui comprend la mise en oeuvre d'un procédé selon l'une des revendications 1 à 3, et l'extraction du lipide contenu dans le corps fongique résultant.

5. Procédé selon la revendication 4, dans lequel le lipide est extrait du corps fongique avec un solvant organique.

6. Procédé selon la revendication 4 ou la revendication 5, dans lequel avant l'extraction du lipide, le mélange résultant de corps fongique et de milieu de culture est déshydraté.

7. Procédé selon la revendication 4 incluant la séparation du corps fongique résultant du milieu de culture.